**Europäisches Patentamt**

**European Patent Office**

**Office européen des brevets**

(19)

(11) Publication number : **0 511 183 A1**

(12) # EUROPEAN PATENT APPLICATION

(21) Application number : **92850088.3**

(22) Date of filing : **22.04.92**

(51) Int. Cl.⁵ : **A61M 5/19**

(30) Priority : **25.04.91 SE 9101261**

(43) Date of publication of application :
**28.10.92 Bulletin 92/44**

(84) Designated Contracting States :
**PT**

(71) Applicant : **Kabi Pharmacia AB**
**Rapsgatan 7**
**S-751 82 Uppsala (SE)**

(72) Inventor : **Hjertman, Birger**
**Svärdsliljevägen 186**
**S-162 43 Vällingby (SE)**
Inventor : **Ljungquist, Olle**
**Havrevägen 21**
**S-183 67 Täby (SE)**
Inventor : **Levander, Gustav**
**Järpstigen 16**
**S-161 28 Bromma (SE)**

(74) Representative : **Onn, Thorsten et al**
**AB STOCKHOLMS PATENTBYRA Box 23101**
**S-104 35 Stockholm (SE)**

(54) **Injection container.**

(57)    An injection container of the dual-chamber cartridge type is formed from two separate coaxial tubular containers, comprising a front container for a first product and a second container for a second product. The front container is closed at its rear end by a fluid-sealing wall, and the rear container is arranged in this wall, and is itself closed at its front end by a fluid-sealing movable wall. In this movable wall are arranged connections, which may be opened by the displacement of said wall, to allow the second product to be transferred into the front container, to be mixed with the first product. Preferably the first product is solid and may be dissolved or dispersed by the second product, which is liquid. Also preferably, the fluid-sealing wall closing the rear end of the front container is also movable, and may be urged forwards to expel the mixture formed in the front container from said container to injection.

FIG.1

EP 0 511 183 A1

The present invention relates to the field of containers for liquids which are to be injected or infused. More specifically, the invention relates to injection containers of the cartridge type, and still more specifically to such containers of the dual-chamber cartridge type.

Injection containers of the cartridge type have found a very wide use for the administration of pharmaceutical and other preparations by injection or infusion. Such cartridges are provided pre-filled with a liquid injectable preparation to the user. One end of the cartridge is adapted to be connected to an injection needle or cannula, and the other end is usually provided with a movable sealing wall, which acts as a piston for expelling the injectable preparation from the cartridge. In use, the cartridge is usually placed in a holder adapted to receive it, and an injection needle or cannula is connected to one end of the cartridge and a plunger or piston rod to the other end. When pressure is applied by the plunger on the movable wall, this wall is forced into the cartridge and the liquid is expelled through the needle, to be administered to a patient, for example.

Cartridges of the type described have a number of important advantages. As they are provided pre-filled with the injectable preparation, there is no need for the user to fill an injection syringe by drawing liquid up from a storage bottle. This decreases the risk of microbial contamination, and greatly simplifies the handling of the necessary equipment when an injection is to be administered.

A further development of the injection cartridges has been the injection cartridge of the so-called dual-chamber type. In such a cartridge, the injectable preparation is divided into two parts which later are to be mixed, for example one liquid and one solid component or two liquids, which are separated in the cartridge by a fluid-sealing, movable wall. The two components are not combined into an injectable preparation until immediately before an injection is to be administered. By this, it has been possible to prepare and inject pharmaceutical preparations wherein the active component is not stable in solution for an extended time.

When the dual-chamber cartridge is to be used for administering a pharmaceutical preparation, pressure is applied on the rear movable wall of the cartridge, such that this wall is urged forwards. As the rear chamber contains the liquid component, which is essentially incompressible, the movable wall separating the solid and the liquid components will also be urged forwards. When the separating wall has been moved forwards a determined distance, one or more bypass channels will be exposed such that the liquid in the rear chamber will flow into the front chamber containing the solid component and dissolve or disperse this component. Such bypass channels are usually arranged in the wall of the cartridge, but may also be arranged in the separating movable wall itself.

After essentially all of the liquid has been made to flow into the front chamber to be mixed with the solid component, the rear movable wall will rest against the separating movable wall, and on further pressure from the rear, the two walls will act together as a piston to expel the solution or dispersion of the solid component in the liquid component from the front chamber through an injection needle or cannula arranged at the front end of the cartridge, to be administered to a patient.

Injection cartridges of the dual-chamber type have found a wide use for injecting pharmaceutical preparations wherein the active component is not stable in aqueous solution or dispersion. Such cartridges are described in the U.S. patents Nos. 2,549,417, 2,607,344, 2,717,601 and others.

However, injection containers of the dual-chamber cartridge type still have a number of shortcomings. Thus, the conventional cartridges are not well adapted to handle relatively large amounts of liquid preparations to be injected. Another, more serious disadvantage is that it has been difficult to achieve complete sterility in the manufacture of such cartridges. The solid component is usually prepared in situ in its chamber of the cartridge by freeze-drying a sterile solution in said chamber. It is possible to sterilize the liquid component in the cartridge by autoclaving at a pressure above atmospheric, but it is not possible to prepare the solid component in situ afterwards by freeze-drying in the cartridge, as the liquid in its chamber will expand under the influence of the low pressure used in the freeze-drying process and will then get into contact with the solid component prematurely. It has therefore usually been necessary to carry out the freeze-drying step first and then fill the liquid component into the rear chamber under aseptic conditions. A final sterilization by autoclaving has not been possible, because of the great instability of the solid component. Thus, the microbial safety of the finished product has left room for improvement.

The shortcomings mentioned above are eliminated to a great extent by the present invention. Through the invention is provided an injection container of the dual-chamber cartridge type which has a greater capacity than that of the prior art cartridges. Also, it has been made possible to manufacture an injection container of the dual-chamber cartridge type with an improved safety against microbical contamination.

According to the invention, an injection container is provided, which comprises a front chamber, which contains a first product, and a rear chamber, which contains a second product, and an openable connection between said front and rear chambers, means to open said openable connection and means transfer to the second product from the rear chamber through said openable connection into the front chamber to get into contact with the first product to be mixed therewith. What characterizes the invention is that the two chambers are formed from two separate coaxial tubular containers, consisting of a front container for the first product and a rear container for the second product, the front container being closed at its rear end by a fluid-sealing wall, wherein is arranged the front end of the rear container, and the rear container is closed at its rear end by a fluid-sealing, movable wall, and at its front end by a fluid-sealing, movable wall, wherein is arranged an openable connection for the second product from said rear container to said front container.

According to the invention, the rear container with its contents has been heat sterilized, preferably by autoclaving, before said two containers have been assembled together.

In the preferred embodiment, the injection container also comprises means for conducting the solution or dispersion formed out from said front chamber to injection, and the front container has a greater diameter than the rear container, such that said rear container may at least partly be introduced into said front container.

The tubular containers usually have a circular cross-section, but this is not strictly necessary, as long as the rear container can be introduced into the front container. Of more importance is that the two containers are arranged essentially coaxial, so that the movement of one container into the other is facilitated.

The movable wall closing the front end of the rear container may be integral with the movable wall closing the rear end of the front container, or it may be separate from said wall.

Preferably, the first product in the front chamber is a solid, and the second product in the rear chamber is a liquid, usually water or an aqueous liquid. The invention will be described in closer detail in the following specification with reference to this preferred embodiment. However, it is to be noted that other embodiments are also possible. Thus, both products may be liquid, and any liquid product may be in the form of a pure liquid, a solution, an emulsion or a suspension. All these embodiments are included in the scope of the present invention.

In the drawings, figures 1-3 schematically show an injection container of the invention and its function. Figures 4 and 5 show another embodiment of the movable wall sealing the rear end of the front container. Figures 6-8 show an injection container of the invention arranged in a holder. Figures 9 to 16 show various embodiments of the invention, wherein means have been arranged to control the sequence of steps carried out for preparing the injectable compostion and administering it by injection. In the drawings, like parts have the same reference numbers. It is also to be noted that the drawings only serve to illustrate the invention and are not intended to limit it in any way.

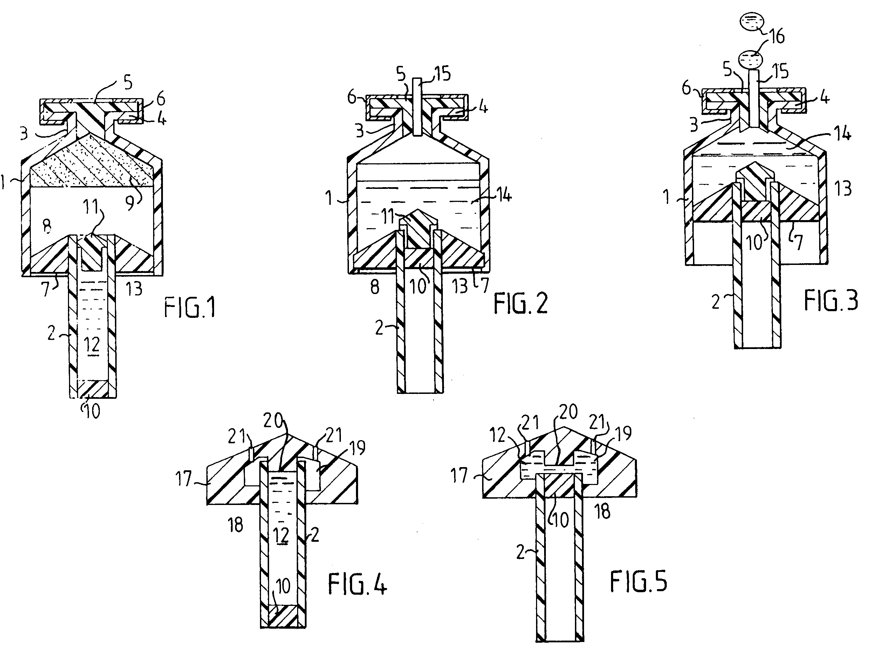

Figure 1 schematically shows an injection container in accordance with the invention. The injection container comprises two coaxial tubular containers, a front container 1, and a rear container 2. The front container 1 has a larger diameter than the rear container 2.

The front container 1 is essentially shaped like a bottle without a bottom, and has a neck 3, which is provided with a flange 4. The opening in the neck 3 is closed by a stopper or septum 5, which can be penetrated by an injection needle or cannula. The septum 5 is held in place by a capsule 6, which secures it by means of the flange 4. The capsule 6 has an opening in its central part to expose the septum 5 for penetration by an injection needle.

At its rear end, the front container 1 is closed by a fluid-sealing movable wall 7, which has a central opening 8, wherein is arranged the front end of the rear container 2. In the embodiment shown in this figure, the rear container 2 is secured in the central opening 8 in such a way that it cannot move in relation to the movable wall 7, which, however, can be moved in relation to the front container 1.

The front container 1 is filled with a solid product 9.

The rear container 2 is closed at its rear end by the fluid-sealing movable wall 10, and at its front end by the fluid-sealing movable wall 11. Between these walls, inside the rear container 2, is arranged a liquid product 12, which is usually water or an aqueous liquid. The front movable wall 11 of the rear container 2 is provided with one or more longitudinal grooves 13, which extend from the rear face of said wall up to a point before the front face of the wall. These grooves serve as bypass channels, and their function will be explained in more detail below.

Figure 2 shows the injection container of the invention when the injectable composition has been prepared. When pressure is applied on the rear movable wall 10 to move it forward, said pressure will be transmitted by the essentially incompressible liquid 12 to the front movable wall 11 to urge it forward. The front movable wall 11 is moved forward until the grooves 13 in it have reached the front end of the rear container 2 with their front ends, so that they are exposed to the interior of the front container 1. A bypass connection will then be established between the interiors of the front and rear containers 1 and 2, and the liquid product in the rear container 2 will flow into the front container 1 when the rear movable wall is moved further forwards. As the pressure in the liquid 12 will decrease when the bypass channels 13 are opened, the front movable wall 11 will not move further forwards, so that it will not be pushed out into the interior of the front container 1. This can also be further assured by the use of suitable mechanical stops, which are not shown in the drawing. Finally, when all the liquid in the rear container has flown into the front container, the rear movable wall 10 will rest against the rear face of the front movable wall 11.

The liquid 12 flowing into the front container 1 will get into contact with the solid product 9 to dissolve it or disperse it, forming the injectable preparation.

Also, an injection needle or cannula 15 has been inserted through the septum 5, This is preferably done before the two products are mixed, as it is then avoided that a pressure above atmospheric is built up in the front container when the liquid is forced into it.

When the solid product 9 has been completely dissolved or dispersed in the liquid product 12 to form a solution or dispersion 14, the container of the invention is ready for injection. This injection is carried out as shown schematically in Figure 3.

To expel the solution or dispersion 14 from the front container 1, pressure is applied on the movable wall 7 of said front container. This wall will then move forwards, and together with the movable walls 10 and 11, it will act as a piston to expel the solution or dispersion 14 through the needle or cannula 15, as is shown by the drops 16. As the rear movable wall 10 rests against the front movable wall 11 in the rear container 2, the bypass channels 13 are closed off at their rear ends, and hence no liquid can flow back through these channels. The front faces of the movable walls 7 and 11 are preferably shaped such that they conform to the shape of the interior front end of the front container 1, so that the dead space after completed injection is minimized.

The needle or cannula 15 may be arranged as a needle pointed at both ends for direct injection from the container. It may also be arranged as a pointed cannula which is connected to a tube, which in its turn is connected to a needle or cannula for administering the preparation to a patient.

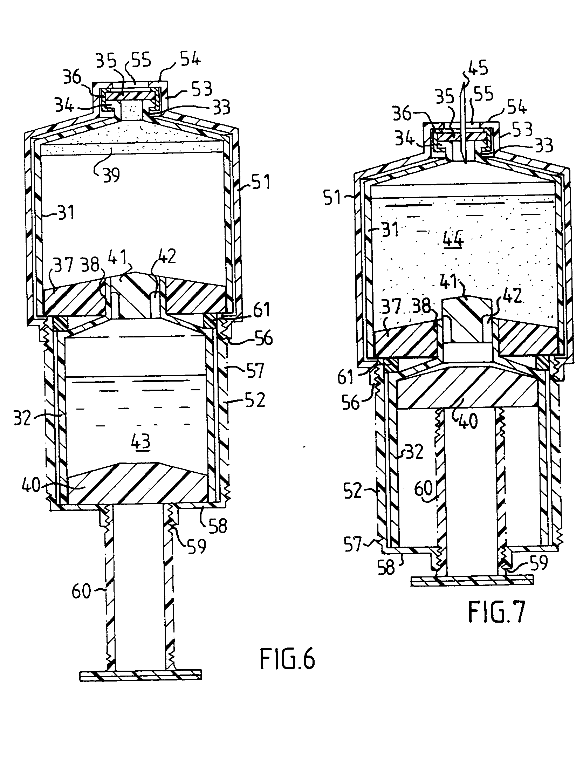

Figures 4 and 5 show another embodiment of the rear movable wall of the front container. Here, the rear movable wall of the front container 1 and the front movable wall of the rear container 2 have been integrated into one single movable wall 17. This wall is provided with a rearward facing opening 18, an interior chamber 19, and a rearward facing projection 20 in the chamber 19. One or more connecting passages 21 are arranged between the interior chamber 19 and the forward face of the movable wall 17. The opening 18 and the projection 20 are arranged such that the rear container 2 will fit fluid-sealingly in the opening 18 at the same time as the projection 20 fits fluid-sealingly in the front opening of the rear container 2.

Before the assembled container has been made ready for injection, the parts are arranged as shown in Figure 4. The front end of the rear container 2 has been inserted through the opening 18 so far that the projection 20 closes the front end of said container. The container has also been filled with the liquid product 12, and is closed at its rear end by the rear movable wall 10. Thus, no liquid can enter the interior chamber 19 and the passages 21. The assembled movable wall 17 has also been inserted into the rear end of the front container 1, as is shown in Figures 1-3. By this arrangement, there is no connection between the outside atmosphere through the passages 21 and the interior of the front container 1, as the rear opening 18 of the wall 17 is closed by the rear container 2.

When pressure is applied to the rear movable wall 10, while container 2 is held fixed, this pressure will be transmitted through the essentially incompressible liquid 12 to act on the projection 20 closing the front end of the rear container 2. As a result, there will occur a relative movement between the rear container 2 and the movable wall 17, such that the projection 20 will be forced out of the front opening of said container 2. On further movement forward of the rear movable wall 10, the liquid product will be forced out into the interior chamber 19 and through the passages 21, to get into contact with the solid product 9 in the front container 1 and dissolve it or disperse it. When all the liquid 12 has been forced out of the rear container 2, the rear movable wall 10 will be in its foremost position, as shown in Figure 5. The container is now ready for injection or infusion in the same way as shown and described for Figure 3.

For a correct function of the device of the invention, it is important that the steps described are carried out in the correct order. Thus, the movable wall 7 may not begin its forward movement until the rear movable wall 10 has been moved fully forwards and all the liquid 12 has been transferred from the rear container 2 into the front container 1. To ensure this correct operating sequence, the container of the invention is usually placed in a suitable holder, which is provided with suitable locking means to prevent the movement of parts out of turn. A number of embodiments using such holders are shown in the following figures. Figures 6 to 8 show a first such embodiment. The injection container comprises a front container 31 and a rear container 32. The front container 31 is bottle-shaped at its front end with a neck 33 and a flange 34. The front end is closed by a piercable stopper or septum 35, which is secured to the flange by a capsule 36. At its rear end, the front container 3 is closed by the movable fluid-sealing wall 37, wherein is arranged the front end of the rear container 32 in a central opening 38. The front container 31 contains the solid product 39.

The rear container 32 is closed at is rear end by the fluid-sealing movable wall 40 and at its front end by the fluid-sealing movable wall 41. The front movable wall 41 is provided with one or more longitudinal grooves 42, which serve as bypass channels for the liquid product 43 when the front movable wall 41 has been moved a certain distance forward, as has been described previously.

The assembled injection container is held in two sleeves, a front sleeve 51, which essentially surrounds the front container 31, and a rear sleeve 52, which essentially surrounds the rear container 32. The front sleeve is provided with a neck 53 and a flange 54, against which rests the flange 34, septum 35 and capsule 36 of the front container 31. At its front end, the front sleeve 51 is provided with a central opening 55, which exposes the septum 35 to an injection needle or cannula.

The rear sleeve 52 is connected to the front sleeve 51 by means of a connection 56, which allows and axial displacement of the rear sleeve 52 in relation to the front sleeve 51. In the Figures 6 to 8, this connection is shown as a threaded connection with threads 57 on the outer surface of the rear sleeve 52 which cooperate with internal threads on the front sleeve at 56. Other means of connection are also possible.

The rear sleeve 52 is closed at its rear end by a wall 58 having a central opening 59 wherein is arranged a plunger 60. This plunger 60 is arranged such that it can be displaced in an axial direction in relation to the rear sleeve 52. Such movement may be a simple sliding movement, or it may be arranged by the use of threads, as is shown in the Figures 6 to 8.

The rear container 32 is held in the rear sleeve 52 by stopping means 61, which may be a ring or a number of projections.

The dimensions of the two sleeves 51 and 52 are such that the assembled injection container fits snugly inside the two said sleeves when they are assembled. The sleeves 51 and 52 and the plunger 60 may be manufactured from any suitable metal or plastic material.

When the injection container is to be readied for injection, it is inserted with its front container 31 into the front sleeve 51, until the flange 34 and septum 35 rest against the inner surface of the flange 54 of the front sleeve 51. The rear sleeve 52 is then placed around the rear container 32 and is secured to the front sleeve 51 by means of the threaded connection 56, 57. At the rear end of the rear sleeve 52, the plunger 60 is inserted into the opening 59 by means of a sliding or threaded engagement, such that the front end of the plunger 60 rests lightly against the rear face of the rear movable wall 40. The complete assembled device is now in the state shown in Figure 6.

When the liquid product 43 is to be mixed with the solid product 39, pressure is applied on the rear movable wall 40 by means of the plunger 60, which is pushed or screwed into the rear sleeve 52. The pressure is transmitted through the liquid 39 to urge the front movable wall 41 forward to a position where the longitudinal grooves 42 are exposed to the interior of the front container 31. These grooves 42 now serve as bypass channels for the liquid 43, so that said liquid 43 flows into said front container when further pressure is applied on the rear movable wall 40. The liquid will now dissolve or disperse the solid product 39 in the front container 31. When all the liquid has been transferred into the front container 31, the device is in the state shown in Figure 7. The rear movable wall 40 is now as close as possible to the front movable wall 41. During this operation, the rear container 32 is prevented from mowing forwards by the stopping means 61. This ensures that the rear container 32 cannot push the movable wall 37 forward prematurely.

An injection needle or cannula 45 has also been inserted through the septum 35. This is preferably done before the liquid 43 is forced into the front container 31, so that the build-up of an overpressure in said front container is prevented.

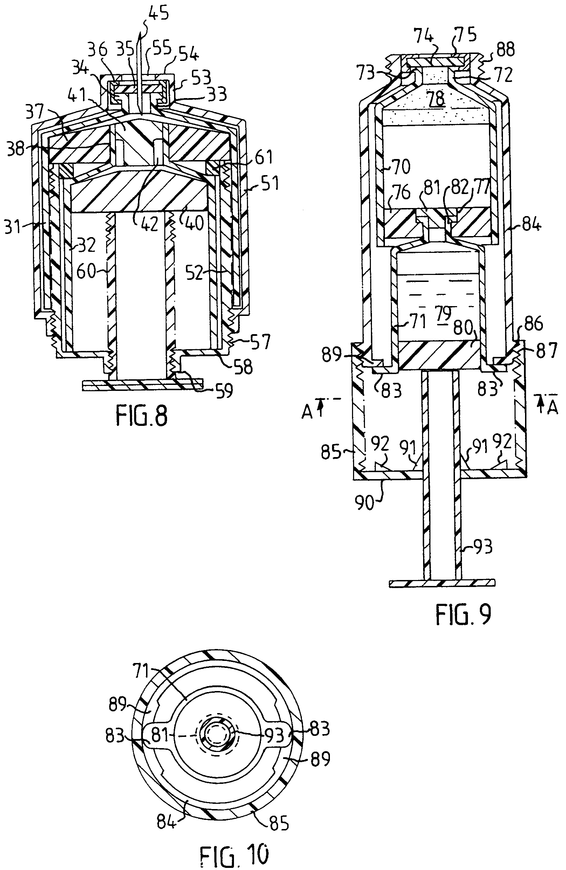

When the solution or dispersion 44 prepared in the front container 31 is to be administered by injection, pressure is applied on the movable wall 37 of said front container, so that said movable wall is urged forwards and the solution or dispersion is forced out through the needle or cannula 45. The pressure on the movable wall 37 may be applied by further movement of the plunger 60, or by movement of the rear sleeve 52 into the front sleeve 51, as shown in Figure 8.

The holder shown in figures 6 to 8 for the injection container is just one example of a suitable holding device, and other embodiments of holding devices are shown schematically in the following figures.

In Figures 9 and 10 is shown a further embodiment of the injection container of the invention. Figure 9 shows a schematic longitudinal sectional view, while Figure 10 is a transversal sectional view along A-A in Figure 9.

The injection container of Figure 9 comprises a front container 70 and a rear container 71. The front container 70 is bottle-shaped at its front end, having a neck 72 and a flange 73. The front end is closed by a piercable stopper or septum 74, which is held in place by a metal capsule 75. At its rear end, the front container 70 is closed by a movable liquid-sealing wall 76, wherein is arranged the front end of the rear container 71 in a central opening 77. The front container 70 contains a solid product 78.

The rear container 71 contains a liquid product 79 and is closed at its rear end by a fluid-sealing movable wall 80, and at its front end by a fluid-sealing movable wall 81. This movable wall 81 is provided with longitudinal grooves 82, which form bypass channels for the liquid product 79 when the front movable wall has been moved a certain distance forward, as has been described in the foregoing. At its rear end, the rear container 71 is provided with at least one and preferably two radially extending projections 83, the function of which will be described in more detail below.

The assembled dual container is enclosed in a front sleeve 84 and a rear sleeve 85, which are releasably joined together by an external thread 86 on the front sleeve which cooperates with an internal thread 87 in the rear sleeve 85.

At its front end, the front sleeve 84 is shaped internally to conform to the neck portion of the front container 70, such that the piercable septum 74 is exposed to be penetrated by an injection needle, which is secured to the front end of the front sleeve 84 by means of the thread 88. At its rear end, the front sleeve 84 is provided with projections 89, which extend radially inward from the internal wall of the front sleeve. These projections 89 do not extend completely around the inner periphery of the front sleeve, but only for a certain distance, as can be seen in Figure 10. The angular spacing of the projections 89 in the front sleeve corresponds with that of the radial projections 83 on the rear container.

At its rear end, the rear sleeve 85 is terminated by a transversal end wall 90, which has a through-going hole 91. On the internal wall of the end wall 90 are arranged lugs 92, the function of which will be described in more detail below.

A plunger 93 is arranged in the hole 91 of the transversal end wall 90. This plunger is provided with a catch or other stopping means 94, to prevent it from being pulled out backwards from the rear sleeve 85.

When this container is to be readied for injection, the front end rear containers 70 and 71 assembled together are first inserted into the front sleeve 84 such that the bottleneck-shaped front end of the front container 71 rests against the matching internal surface of the front sleeve 84. The septum 74 will then be exposed through the opening at the front end of the front sleeve 84, so that it can be pierced by an injection needle.

When the front and rear containers are inserted into the front sleeve 84, they should be positioned such that the outwardly directed radial projections 83 at the rear end of the rear container 71 rest against the radial inward projections 89 at the rear end of the front sleeve 84. This will prevent the rear container 71 from being moved forward prematurely.

The rear sleeve 85 with the plunger 93 is then screwed onto the front sleeve 84 until the front end of the plunger 93 rests lightly against the rear surface of the rear movable wall 80 of the rear container 71. The device is now ready for the mixing of the two components in the front and rear containers.

When this mixing is to be carried out, the rear sleeve 85 is screwed further onto the front sleeve 84. As the plunger 93 cannot be moved backwards because of the stopping means 94, this will cause the front end of the plunger 93 to push the rear movable wall 80 forward in the rear container, building up a pressure in the liquid 79 in said rear container. This pressure will cause the front movable wall 79 to be moved forward, such that the longitudinal grooves will afford a liquid connection with the interior of the front container 70, and the liquid will flow into this front container to dissolve or disperse the solid component 78.

When the rear sleeve 85 has been screwed fully home on the front sleeve 84, the rear movable wall 80 has also been pushed to its foremost position in the rear container 71, and essentially all the liquid 79 has been transferred into the front container 70. The device is now ready for the administration of one or more injections.

Before or after the two components have been mixed, an injection needle or cannula has been attached to the front end of the front sleeve 84, by means of the thread 88, so that a liquid connection is established with the interior of the front container 70. This injection needle or cannula is of a conventional design, and does not have to be described in closer detail.

When the rear sleeve 85 has been screwed fully home on the front sleeve 84, the lugs 92 on the front face of the transversal rear wall 90 are situated on the same level as the outward radial projections 83 at the rear end of the rear container 71. A further turning of the rear sleeve 85 by a small amount, less than one complete turn, will make the lugs 92 act on the outward radial projections 83 such that the rear container 71 is rotated around its longitudinal axis to move the outward radial projections 83 out of engagement with the inward radial projections 89 at the rear end of the front sleeve 84. The rear container 71 is now no longer prevented from being moved forward. This forward movement is effected by pushing the plunger 93 forward, whereby the rear container 71 and the rear movable wall 76 of the front container 70 are moved forward, and the mixture of the two components in the front container 70 is expelled therefrom through the attached injection needle or cannula. The injection may be administered with the complete contents at one time, or it may be divided into a number of injections. In the latter case, the injection needle is usually removed an discarded after each injection, and a fresh needle is attached. The injection may also be administered over an extended time. In such cases, the cannula from the front container is usually connected to a tube, which in its turn is connected to an injection needle for administering the injectable preparation to the patient.

After the desired amount has been administered, the rear sleeve 85 may be unscrewed from the front sleeve 84, and the combined containers 70 and 71 taken out and discarded. A fresh assembly of a front and rear container may then be inserted, and the whole procedure for mixing and administering may be repeated as many times as desired. As the injectable preparation will not come into contact with the front and rear sleeves 84 and 85, it should not be necessary to sterilize these sleeves between each use.

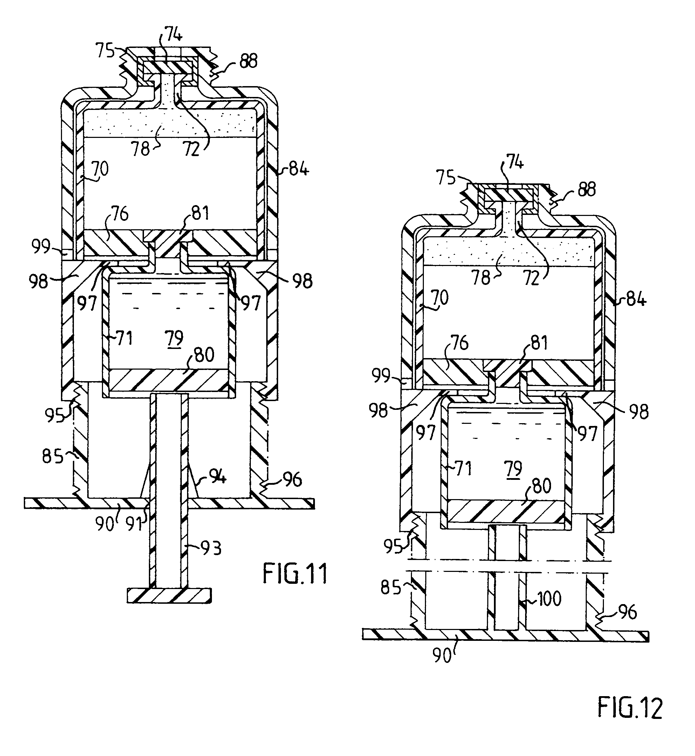

A number of embodiments of the device of the invention are possible to maintain the correct sequence of operations to ensure that the components in the two containers are thoroughly mixed before any injection can take place. A number of such embodiments are illustrated in Figures 11 to 16:

Figure 11 shows a variant of the embodiment shown in Figure 9 and 10. The device for injection shown here also comprises a front container 70 and a rear container 71, containing a solid product 78 and a liquid product 79, respectively. At the front end of the front sleeve 84, an arrangement is made for attaching an injection needle or cannula by means of the thread 88, to pierce the septum 74, as previously described. At the rear end of the rear sleeve is arranged a plunger 93 through a hole 91 in the transversal rear end wall 90 of the rear sleeve 85, said plunger being provided with stopping lugs 94.

The liquid-sealing movable walls 76, 80 and 81 of the front and rear containers, respectively, are arranged in the same way as described for Figures 9 and 10.

In the embodiment shown in this Figure, the front sleeve 84 is provide with an internal thread 95, and the rear sleeve 85 is provided with an external thread 96. The external thread 96 runs for the whole length of the rear sleeve 85, while the internal thread 95 of the front sleeve 84 only has to run for a short length from the rear end forwards.

In the front sleeve 84 are arranged inwardly directed projections 97, which are situated at such a level that they extend inwards between the shoulders of the bottle-shaped rear container 71 and the rear movable wall 76 of the front container 70. The projections 97 merge into the wall of the front sleeve 84 via an inclined part 98, and are made resilient, such that they can be bent outwards from the front sleeve. This resiliency may for instance be achieved by making longitudinal slits in the wall of the sleeve on each side of each projection and a peripheral slit 99, and by selecting a suitable material for the front sleeve, such as a suitable plastic material. The projections 97 only extend for a short distance in the peripheral direction, and are arranged with a regular spacing around the inner periphery of the front sleeve 84.

When the device is to be readied for injection, the rear sleeve 85 is screwed into the front sleeve 84 by means of the thread 95, 96. At the same time, the front end of the plunger 93 will push the rear movable wall 80 of the rear container 71 forward, so that the front movable wall 81 is displaced and allows the liquid 79 to flow into the front container 70, to be mixed with the solid product 78, as previously described.

Through the projections 97, the rear container 71 is prevented from being moved forward at the same time as its rear movable wall 80 is pushed forward by the plunger 93.

When the rear sleeve 85 has been screwed into the front sleeve 84 so far that all the liquid in the rear container 71 has been transferred into the front container 70, a further movement forward by the rear sleeve 85 will make its front edge press against the inclined parts 98, and this pressure will move the projections 97 outward in the radial direction, such that they no longer prevent the rear container 71 from being moved forward, Thus, the rear container 71 and the rear movable wall 76 of the front container 70 may now be pushed forward by actuating the plunger 93, to expel the mixture of the solid and liquid products from the front container through an injection needle or cannula, which has been connected through the septum 74 to the front container 70 at a suitable time, as described in the foregoing.

This embodiment has the advantage that it will not be necessary to subject the containers to any rotating movement to free the rear container before the mixed preparation is to be injected.

Figure 12 shows a still further embodiment of the device shown in Figure 11. This embodiment is the same as that shown in Figure 11, but the plunger in Figure 11 has been replaced by a projection 100, which extends forward from the front face of the rear transversal wall 90. This projection may be tubular, as shown in the Figure, or it may be rod-shaped.

When the device is to be readied for injection, the rear sleeve 85 is screwed into the front sleeve 84, whereby the projection 100 will push the rear movable wall 80 of the rear container 71 forward to effect mixture of the liquid and solid products, such as previously described. However, when the rear sleeve has reached the position where it urges the projections 97 outwards, by means of the inclined parts 98, the rear sleeve is screwed further into the front sleeve to administer the injection. The projection 100 will then push the rear container 71 and the rear movable wall 76 of the front conatiner forward, so that the mixed preparation formed in the front container is expelled through an injection needle or cannula, which has been attached to the front end of the front sleeve 84, as described in the foregoing.

It is to be noted that to show the function of a number of working parts in the figures of the drawings more clearly, said parts are not drawn to scale, but are often exaggerated.

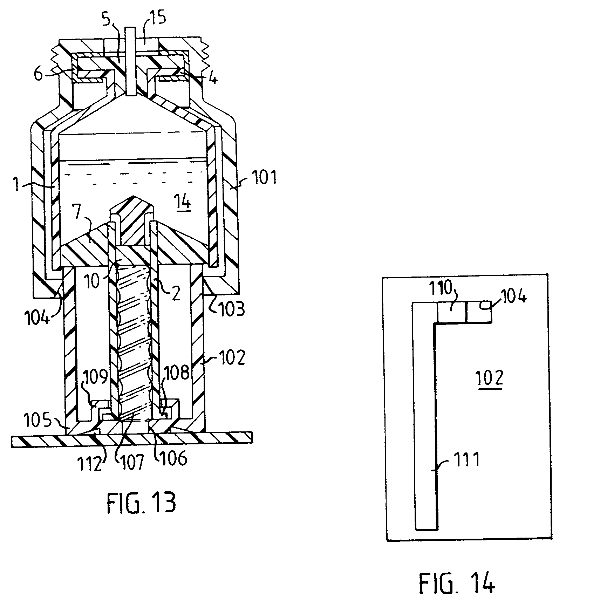

Another embodiment of the device of the invention is shown in Figures 13 and 14. It will be seen that this is essentially the device schematically shown in Figure 2 which has been enclosed in a front sleeve 101 and a rear sleeve 102, with the front sleeve being adapted to receive an injection needle at its front end, as described previously. The front and rear sleeves are held together by inwardly directed radial projections 103 at the rear end of the front sleeve, which lock in matching depressions 104 in the wall of the rear sleeve 102 such that any axial movement between the two sleeves is prevented. This arrangement will be described in more detail below.

At its rear end, the rear sleeve 102 is terminated by a transversal end wall 105, which has a threaded hole 106, which accepts a threaded piston rod 107.

When this piston rod 107 is screwed into the rear sleeve 102, its front end will push the rear movable wall 10 of the rear container 2 forward.

The rear end of the rear container 2 is provided with outwardly extending radial projections 108, which cooperate with axially extending catches 109 from the inside of the transversal end wall 105 to form a bayonet lock. By this arrangement, the rear container 2 is retained in place when the rear movable wall 10 is pushed forward by the piston rod 107.

Figure 14 shows schematically the holding arrangement in the wall of the rear sleeve 102. The depression 104, wherein rests the projection 103 of the front sleeve (not shown) is delimited on one side in the peripheral directions by a resilient projection 110, on the other side of which an axial groove 111 extends towards the rear end of the rear sleeve 102. This groove also accepts the projection 103 of the front sleeve, such that when said projection 103 is in the groove 111, an axial movement between the front and the rear sleeve becomes possible.

When this device is to be readied for injection, the piston rod 107 is screwed into the hole 106 of the transversal end wall 105, to push the rear movable wall 10 of the rear container 2 forward. This will make the liquid product 12 in the rear container 2 flow over into the front container 1, to be mixed with the solid product 9, as previously described. During this movement, the rear end of the rear container 2 is secured to the inside of the transversal end wall 105, so that it cannot follow the movable wall 10 forward in its movement. Also, the front and rear sleeves are locked together by the projections 103 locking in the depressions 104 on the respective sleeves, so that the rear sleeve cannot push the movable wall 7 forward.

When the piston rod 107 has been screwed fully home, such that all the liquid in the rear container has been transferred into the front container 1, the device is ready for the administration of injections. For this, the rotation of the piston rod is continued in the same direction, whereby lugs 112 arranged on a handle at the rear of the piston rod 107 will engage the rear sleeve 102 to make it follow the rotation for a short distance. During this continued rotation, the projections 103 at the rear end of the front sleeve 101, which are originally resting in the depressions 104, will be forced to override the resilient projections 110, to get into the longitudinal grooves 111. At the same time, this continued rotation may also release the bayonet lock 108, 109 at the rear end of the rear container 2, but this is not strictly necessary, as the rear end will follow the rear sleeve 102 forward in its movement anyway.

As the radial projections 103 of the front sleeve 101 are now resting in the longitudinal groove 111 in the wall of the rear sleeve 102, it is now possible to push the rear sleeve 102 forward in relation to the front sleeve 101. This movement will also urge the rear container 2 and the movable wall 7 of the front container 1 forward, so that the mixed preparation in the front container will be expelled through the needle 15.

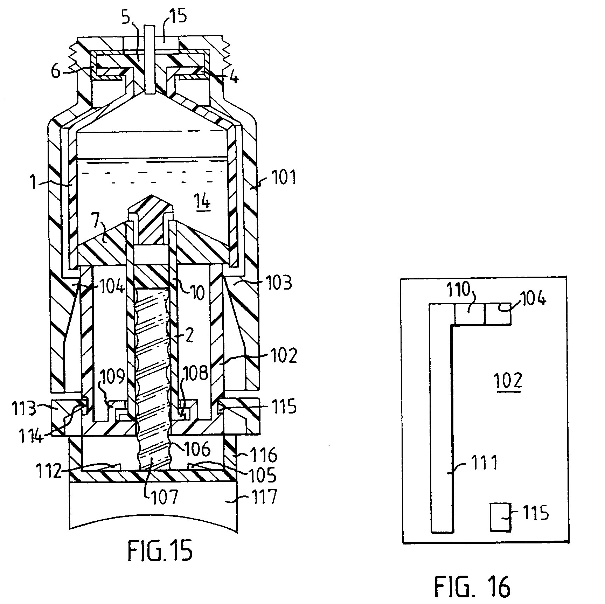

The embodiment shown in Figures 15 and 16 is essentially the same as that shown in Figures 13 and 14, but further safety means have been added against a premature axial movement of the rear sleeve in relation to the front sleeve.

The device comprises a front container 1 and a rear container 2 enclosed in a front sleeve 101 and a rear sleeve, and a threaded piston rod 107 through a correspondingly threaded hole 106 in a transversal rear end wall 105 of the rear sleeve 102. The front and rear sleeves 101, 102 are held together by the radially extending projections 103 resting in the depressions 104 in the wall of the rear sleeve 102. In this embodiment, however, the front sleeve is extended backwards to include further radially inward-extending projections 113, which lock in depressions 115 in the wall of the rear sleeve 102. These projections 113 are resilient and have inclined parts 114 facing rearward.

The piston rod is also provided with a short tubular part 116 extending forward from a handle 117 of said piston rod. The diameters of the front sleeve 101, the rear sleeve 102 and the tubular part 116 are so adapted to each other that the tubular part 116 fits between the rear sleeve 102 and the front sleeve 101.

Figure 16 shows a schematic view analogous to Figure 14 of a part of the side of the rear sleeve 102. In the same way as in Figure 14, there are provided a depression 104, a resilient projection 110, and a longitudinal groove 111. There is also a further depression 115, wherein the projection 113 of the front sleeve 101 is intended to rest.

When the device is to be readied for injection, the piston rod 107 is screwed into the hole 106 of the transversal end wall 105, to push the rear movable wall 10 of the rear container 2 forward to effect mixing of the two components as described in the foregoing. Through the bayonet lock arrangement 108, 109 at the rear end of the rear container and the radial projections 103 and 113 from the front sleeve 101 locking into the depressions 104 and 115, respectively, in the outer wall of the rear sleeve 102, no relative movement is possible between the front and the rear containers 1 and 2, and between the front and rear sleeves 101 and 102.

When the piston rod has been screwed fully home, so that all the liquid product from the rear container 2 has been transferred to the front conatiner 1, the tubular part 116 extending forward from the handle 117 will abut against the inclined parts 114 of the resilient projections 113, such that these projections are urged out of the depressions 115 in the wall of the rear sleeve 102. Further turning of the piston rod 107 will then release the projections 103 from their corresponding depressions 104 in the wall of the rear sleeve 102, so that these projections now lie in the longitudinal groove 111, such as has been described in the foregoing. The piston rod together with the rear sleeve 102 can now be pushed forward to move the movable wall 7 forward to expel the mixed preparation from the front container, as has been described previously.

In all the previously described embodiments of the invention, the rear container has been arranged to be pushed into the front container together with the movable wall sealing the rear end of the front container. This, of course, has necessitated that the rear container has a smaller diameter than the front one. However, this embodiment is only necessary when it is desired to use the device of the invention for first mixing the components and subsequently administering an injection or infusion directly from the front container by means of an attached needle or cannula. However, it is also possible to use the device of the invention solely for mixing the components of the preparation, and subsequently draw off the mixed preparation from the the front container, for example by using a conventional syringe, the needle of which is penetrating a septum arranged to close off the front container from the atmosphere. In such an embodiment, the wall closing the rear end of the front container may be fixed, with the openable connection between the front and rear containers arranged in it. Also, the arrangement of front and rear sleeves may be simplified, as it is no longer strictly necessary, although still desired, to prevent any movement of the rear wall of the front container before all of the liquid component has been transferred from the rear container to the front one. The further changes and modifications which may be necessary or desirable for this embodiment of the invention, are easily understood by persons skilled in the art. The advantages previously pointed out for the invention will be achieved also with this embodiment.

The injection device of the invention is fabricated by conventional process steps, which are easily understood by those skilled in the art. Also the selection of materials to be used for the various parts of the device is within the competence of an expert in this field.

Through the present invention, it has been made possible to provide an injection container of the dual-chamber cartridge type, which has a number of advantages which have not been attainable up to now. A person skilled in the art will also understand that the embodiments specifically shown in the drawings and described in connection therewith are only examples, and do not serve to limit the invention in any way. Thus, modifications and variations of the invention are possible within the scope of the appended claims. For example, though reference has been made previously in the specification to tubular or cylindrical containers and sleeves, this does not mean that the invention is restricted only to containers and sleeves having a cirkular cross-section. Other cross-sections are also possible, provided that the parts in question have a common center axis.

Furthermore, the invention is not limited to the use of only two containers, a front and a rear one, but three or more containers arranged consecutively are also possible. Although such an apparatus is more complicated, its construction and function will be understood by persons skilled in the art.

## Claims

1. An injection container, comprising at least a front chamber, which contains a first product, and a rear chamber, which contains a second product, and an openable connection between said front and rear chambers, and means to open said openable connection and to transfer the second product from the rear chamber through said openable connection into the front chamber to get into contact with the first product to be mixed therewith, characterized in that said two chambers are formed from two separate, coaxially arranged tubular containers, the front container being closed at its rear end by a fluid-sealing wall, wherein is arranged the front end of the rear container, and that the rear container is closed at its rear end by a fluid-sealing, movable wall, and at its front end by a fluid-sealing, movable wall, wherein is arranged said openable connection for the second product from said rear container into said front container, and that the rear container with its contents has been heat sterilized before said two containers have been assembled together.

2. Injection container according to claim 1, characterized in that the first product is solid and the second product is liquid, said solid product being soluble or dispersible in said liquid product.

3. Injection container according to claim 1 or 2, characterized in that the second product is water or an aqueous liquid.

4. Injection container according to any of claims 1-3, characterized in that the movable wall closing the front end of the rear container is integral with the wall closing the rear end of the front coantiner.

5. Injection container according to any of claims 1-3, characterized in that the movable wall closing the front end of the rear container is separate from the wall closing the rear end of the front container.

6. Injection container according to any of claims 1-5, characterized in that the movable wall closing the front end of the rear container is provided with passages which are opened when said movable wall is moved forwards in relation to said rear container, to conduct said second product into the front container.

7. Injection container according to any of claims 1-6, characterized in that the fluid-sealing wall closing the rear end of said front container is movable, and that the rear container has a smaller diameter than the front container, such that it can be moved forward into said front container together with the fluid-sealing movable wall closing the rear end of said front container, to expel the mixture of said first and second products from said front container through a conduit arranged therein.

8. Injection container according to claim 7, characterized in that said first and second containers are arranged as an assembly in a holding device, which is provided with locking means for preventing that the rear container is moved forward into the front container before the second product in said rear container has been transferred into said front container.

9. Injection container according to claim 8, characterized in that said holding device comprises a front sleeve, enclosing the front container, and a rear sleeve, enclosing the rear container, and a piston rod for urging the fluid-sealing movable wall closing the rear end of the rear container forward to transfer the second product into the front container, said locking means being arranged between said two sleeves.

10. Injection container according to claim 9, characterized in that said locking means comprise one or more radially extending projections arranged on the front sleeve, said projections barring releasably the forward movement of the rear container.

11. Injection container according to claim 9, characterized in that said locking means comprise at least one radially extending projection arranged on the front sleeve, said projection or projections locking releasably in one or more corresponding depressions arranged in the wall of the rear sleeve.

12. Injection container according to any of claims 1-6, characterized in that the fluid-sealing wall closing the rear end of the front container is essentially fixed, such that the mixture formed by said first and second products in the front container is drawn off from said container by introducing external means for this into said container.

FIG.1

FIG.2

FIG.3

FIG.4

FIG.5

EP 0 511 183 A1

FIG.6

FIG.7

FIG.8

FIG. 9

FIG. 10

FIG.11

FIG.12

FIG. 13

FIG. 14

FIG.15

FIG. 16

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl⁵) |
|---|---|---|---|
| X | DE-A-1 909 794 (FA. EHRHARDT-SÖHNE) * Page 6, line 11 — page 9, line 19; figures 1-5 * | 1-4, 6-7 | A 61 M 5/19 |
| X | US-A-3 838 689 (COHEN) * Figures 1-3 * | 1-4, 6-7 | |
| X | GB-A-2 229 374 (BESPAK PIC) * Page 3, line 31 — page 6, line 11; figure 1 * | 1-3, 5-7 | |
| X | US-A-4 153 057 (KÖBEL) * Column 2, line 37 — column 3, line 48; figures 1-5 * | 1-3,5, 12 | |
| A | US-A-4 464 174 (ENNIS) * Column 8, line 58 — line 64; figure 1 * | 8-11 | TECHNICAL FIELDS SEARCHED (Int. Cl⁵) A 61 M |
| A | SE-B-324 209 (NOVO TERAPEUTISK LABORATORIUM AS) * Claim 1 * | 8-11 | |

The present search report has been drawn up for all claims

| Place of search | Date of completion of the search | Examiner |
|---|---|---|
| STOCKHOLM | 13-06-1992 | HALLNE M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82